# EUROPEAN PATENT APPLICATION

(11) **EP 1 543 835 A1**
(43) Date of publication of application: **22.06.2005**
(21) Application number: 02747728.0
(22) Date of filing: 01.07.2002
(51) Int. Cl.: A61K 39/00, G01N 33/564

(54) **METHOD OF TRANSFORMING AUTOIMMUNE-DISEASE-CAUSING HAPTEN-ANTIGENS (HAPIGENS) OF AN ORGANISM INTO COMPLETE ANTIGENS**

(71) Applicant: SUAREZ MENDOZA, Ramon, Morelia, Michoacan 58000 (MX)
(72) Inventor: SUAREZ MENDOZA, Ramon, Morelia, Michoacan 58000 (MX)
(74) Representative: Atkinson, Peter Birch
(86) International application number: PCT/MX2002/000061
(87) International publication number: WO 2004/002522

(57) **Abstract**

The invention relates to the field of immunology and, more specifically, to a method of transforming the hapigens of a sick organism into complete antigens. According to the invention, a blood sample is taken and broken down as much as possible without being denatured, i.e. all of the properties thereof are retained as a living organism. The blood sample is subsequently diluted in an isotonic liquid and, consequently, evasive mutant infections germs such as viruses, streptococcus, staphylococcus and bacteria are converted into complete antigens which the treated sick organism can neutralise. An autovaccine is administered to a human patient in order to relieve the symptoms of autoimmune diseases and allergic ailments, such that complete immunity to the ailment is achieved.

## Description

### Field of the invention

The present invention relates to the field of immunology and, more specifically to incomplete immunity reactions such as allergies and diseases which, until now, have been called autoimmune diseases; by the administration of minimal doses of desensitising extracts, broken down as much as possible, of the very substances that cause the disorder.

### Background to the invention

### Immunisation

A technique in preventative medicine, of which the aim is to confer immune resistance to an infectious organism.

For this purpose, an individual is inoculated with a form of the pathogenic organism which is capable not of producing the disease but of inducing the formation of antibodies. This process is also known as vaccination since the first immunisation technique involved the administration of the cowpox virus to achieve immunity to smallpox. Vaccines are the most effective form of protection against viruses and other organisms against which antibiotics are ineffective. In Western countries, specific vaccines are administered in accordance with an official vaccination calendar. Diphtheria, tetanus and whooping cough vaccines are administered simultaneously at the age of 3, 5 and 7 months, coinciding with the poliomyelitis vaccine; the mumps, measles and rubella (triple virus vaccine) vaccines are administered at 15 months. The haemophilus B vaccine is also being given more and more routinely.

The vaccines are prepared using microorganisms (Dr Salk) which are killed by exposure to heat or to chemical agents (such as the first polio vaccine or the typhoid fever vaccine); with a toxoid, which is an inactivated form of the toxin produced by the microorganism (tetanus and diphtheria vaccines) or with an attenuated live virus, in other words a virus which has been weakened in the laboratory so that it does not produce the disease (such as the polio vaccine developed by Albert Sabin or the measles and yellow fever vaccines).

The British doctor, Edward Jenner, performed the first modem immunisation in 1796, by inoculating the cowpox virus to obtain an immune response to smallpox. In 1885, the French scientist Louis Pasteur was the first to use an attenuated virus, the rabies virus, to achieve immunisation against the natural infection. In 1897, a vaccine against typhoid fever was developed in England.

The immunising preparation is introduced into the organism through the skin (inoculation), except for some exceptions such as the Sabin type oral polio vaccine. The duration of the protective effect is highly variable, from 6 months in the case of the plague to 10 years for yellow fever.

There are 2 different vaccination strategies for immunising a population: selective vaccination only of those individuals with the greater probability of suffering the disease, or the principle of immunity as a whole, which is usually adopted: if the probability that an individual with a certain infectious disease will come into contact with a susceptible individual (without immunity to this microorganism) is very low in a population, transmission of the disease tends to disappear. It is not necessary to vaccinate all of the population, but levels of protection of at least 90% of its members must be reached in the case of many diseases. This strategy, or mixed forms, is the most widely used in developed countries. In the case of rubella, for example, the health authorities supervise the vaccination of all school children and of women of a fertile age.

Work is still being carried out to improve or create new vaccines: hepatitis B, hepatitis C, more effective, painless rabies vaccines, or vaccines against the main causes of pneumonia. Vaccines against cholera, or against parasitic infections such as malaria or Tripanosomiasis are being investigated in the developing countries. In addition to active immunisation, which forms the basis of the large majority of vaccines (induction of the production of antibodies by inoculating a form of the infectious organism), another method of conferring resistance to infection is through passive immunisation (administration of a serum which already contains these antibodies because it has been obtained from a person who has previously suffered from the disease). Passive immunisation is only used on rare occasions, such as in some cases of hepatitis.

### So-called autoimmune diseases

Diseases in which the immunological system reacts to components of the organism (proteins) as if they were foreign substances, either by producing antibodies or by another type of reaction

B lymphocytes (a type of leukocyte) produce antibodies. Genetically, a human being possesses approximately 100,000 different classes of antibody which are capable of reacting to an equivalent number of protein substances. During development of the embryo, lymphocytes capable of reacting against the proteins of the organism itself are inactivated so that innate proteins can be distinguished from foreign proteins, and the destruction thereof by the immunological system is avoided. Auto-reactive lymphocytes (hapigens: foreign bodies which are only partially antigenically active) are found in some adults, and this suggests that they are partially repressed in their active form and are not eliminated. Another possible mechanism for protecting innate proteins is based on sequestration (separation) during early development. For example, spermatozoids do not appear until the immunological system has matured; they are separated from the bloodstream at any moment and, therefore, from the reach of the immunological system. After a vasectomy, these cells come into contact with the blood, where they can cause the formation of anti-spermatozoidal antibodies.

A hypothesis explaining the phenomenon of autoimmunity proposes that the suppression of the reaction against innate proteins is impaired when specific viruses infect the antibody-generating cells. In infectious mononucleosis, the lymphocytes are invaded by the cytomegalovirus and appear in the bloodstream as antibodies against various organic proteins. The cardiopathy of rheumatic fever is the consequence of infection of the pharynx or tonsil by bacteria of the Streptococcus genus; at their surface, these bacteria have a protein which is very similar to another protein of the muscle and the cardiac valves; and the antibodies developed against Streptococcus are also capable of attacking and damaging cardiac tissue.

At present, the reason for the formation of autoantibodies is not known for the majority of so-called autoimmune diseases. Patients with myasthenia gravis have antibodies which block neuromuscular transmission; this causes muscular weakness and respiratory problems. In autoimmune haemolytic anaemia, the antibodies destroy red blood cells (red corpuscles). Patients with lupus erythematosus produce antibodies against various innate cell components, including genetic material; antibody-protein compounds aggregate, forming large bodies which may damage the kidneys when they are excreted. In 80% of patients with rheumatoid arthritis, the blood contains an antibody known as the rheumatoid factor; it is not known whether this is what causes the destruction of the articular cartilage which characterises the disease.

The most important group of so-called autoimmune diseases are diseases of the collagen: lupus erythematosus, rheumatoid arthritis, scleroderma and dermatomyositis. In these diseases, the autoantibodies predominantly affect the connective tissue, the main protein of which is collagen. The lesion is very significant in the collagen of the blood vessel walls.

Type I diabetes (which especially affects children and young people) is produced by an antibody which destroys the beta cells of the Langerhans' islets of the pancreas (which produce insulin). Autoimmune chronic thyroiditis is caused by antibodies against the thyroid tissue. Any cases of Addison's disease are produced by immune destruction of the suprarenal capsules.

Multiple or systemic sclerosis is one of the most widely studied so-called autoimmune diseases. A type of pathological lymphocyte destroys the myelin sheath covering the axons of the neurons of the central nervous system. It is not mediated by antibodies. The progressive deterioration produces multiple neurological signs, including blindness or paralysis.

Autoantibodies are frequently found in the blood of formerly healthy people: there is no known explanation for this phenomenon.

The treatment of so-called autoimmune diseases involves immunosuppression, generally by means of steroids, although other pharmaceuticals such as methotrexate are also used. Nowadays, plasmapheresis, a technique in which the patient's blood is passed through an extemal system that eliminates gammaglobulins, which is the antibody-containing fraction of the blood proteins, is also being tested.

### Acquired immunity

The capacity to respond specifically to foreign matter is acquired by interaction with the antigens (foreign substances which are potentially dangerous to the organism) present in such organisms. Invertebrate animals have little or no capacity to respond in a genuinely specific manner; acquired immunity reaches its maximum development in birds and mammals. Amphibians and fish have some acquired immune capacity and primary vertebrates, such as the lamprey, are capable of responding to specific antigens, although the response is usually weak and relatively simple.

Acquired immunity is based on the activity of two systems: the humoral system and the cell-mediated system. Humoral immunity is mediated by soluble proteins known as immunoglobulins or antibodies. Mammals produce five different classes of immunoglobulin molecule known as G, M, A, D and E. Parts of the structure of these relatively large globular protein molecules consist of short sequences located very close to the surface of the molecule, which interact specifically with the antigen. Other parts of the antibody molecules act as intermediaries in immunological functions such as the interaction of activation of the complement with macrophages and other cells.

All classes of immunoglobulin are present in the blood, but immunoglobulin G (IgG) is the dominant class and is also an important serum protein (the serum of normal human beings contains between 8 and 16 mg/ml of IgG). The IgM antibodies are induced in the blood in the first phases of the immune response and IgA are secreted in the gastric and pulmonary fluids, in the perspiration and in the saliva. The IgE mediates the anaphylactic (histamine-liberating) and allergic responses and may be important in providing protection against parasites. The function of circulating IgD is not well known. The immunoglobulins produce them and secrete the B lymphocytes.

The antibodies are specifically linked- to foreign organisms and substances. This frequently causes deactivation of undesirable properties. The antigen-antibody complexes are eliminated from the body by various processes, and the microorganisms covered with antibodies are particularly sensitive to phagocytosis on the part of the macrophages and other cells. After interacting with an antigen, the antibodies activate a range of immunobiological mechanisms which provide protection against infections and other undesirable effects.

Cell-mediated immunity is channelled through the T lymphocytes. Antigen-specific T cells are produced and interact with the antigens to mediate a series of immunobiological functions. An example of this device is the production of cytotoxic cells which specifically destroy undesirable microorganisms or cells. A class of T lymphocytes known as killer lymphocytes or assassin or suppressor cells also destroy foreign cells and microorganisms. The acquired immune response complements the innate immune response and therefore configures a very effective specific system.

### Control of acquired immunity development

The acquired immune system is subject to rigorous control. The B lymphocytes which secrete antibodies are greatly influenced by the T cells which can assist or suppress the response. These T cells secrete cytokines and other potent, biologically active molecules which cause or inhibit activation, maturation and capacity to secrete appropriate immunoglobulins from the B cells. These B cells (and cells of other types) also secrete immunity-modulating cytokines. In particular, the interleukins 1, 2, 4, 6, 10, 12, 13, 14, 15 and 16, interferon g and the transforming growth factor β influence the development and modulation of the acquired immune response. IL-12 is a potent stimulator of the suppressor cells.

### Immunological diversity

The acquired immune system is capable of producing antibodies and T cells which identify a very large number of different molecules with notable specificity. It has been estimated that mammals can produce about 1 million different antibodies, and the mechanism capable of generating this diversity has been the main object of immunological investigation. It is also known that antibodies can be produced against synthetic substances which are not found in nature. "Early theories which attempted to explain this diversity considered that the basis of specificity was a "teaching" process which included "induced adjustment" of antibodies with antigens".

This theory has been largely abandoned since no convincing biological basis to support it has been conceived or discovered. It is currently known that immunoglobulins are produced by reorganisation and union of various genes and that somatic mutation of genetic materials occurs during the development of acquired immunity. This predominantly affects the very variable portions of the immunoglobulin protein which shapes the point of identification of the antigen.

These processes perform an important function in creating diversity and specificity in the immune system. The development of humoral immunity is dependent on the interaction of antigens with IgM or IgD molecules present at the surfaces of the B cells. This initiates activation of the B cells which, if they have sufficient help from the T cells or other systems, causes maturation of the response and the secretion of appropriate antibodies. This idea was proposed by the German immunologist Paul Ehrlich, several decades before it was demonstrated by experiments. The T cells interact with antigens via T cell receptors which have structural morphological similarities to immunoglobulins.

### Paper in the investigation

Biologists have concentrated on the molecular study of viruses and their interaction with the host cell. Investigation of the replication of bacteriophages in bacteria revealed the existence of messenger RNA bearing the genetic code of the DNA required for the synthesis of proteins. Studies of these viruses have also been instrumental in defining the biochemical factors which initiate and terminate the use of genetic information. Knowledge of the mechanisms controlling viral replication is fundamental to understanding biochemical events in superior organisms.

Viruses are useful as model systems for studying the mechanisms controlling genetic information since, in essence, they are small pieces of this information. This enables scientists to study replication systems which are more simple and manageable but which function by the same principles as those of the host cell. The investigation into viruses aims predominantly to discover their replication mechanism, in order to find a method of controlling their growth and eliminating viral diseases. Studies of viral diseases have contributed enormously to an understanding of the immune response of the organism to infectious agents. Studies of this response disclose in depth the serum antibodies and the secretions of the mucous membranes which help the organism to eliminate foreign elements such as viruses. Scientific interest is now focusing on attempts to isolate specific viral genes. These may be cloned to produce large quantities of specific proteins, which would be used as vaccines for diseases such as herpes.

Herpes (from the Greek "herpein", to creep) is the generic name of various types of cutaneous eruption caused by the most significant pathogenic human viruses. Their main representatives are herpes simplex virus type 1 or type 2 and varicella-zosta. Other significant herpes viruses are the Epstein-Barr virus, which causes infectious mononucleosis, and the cytomegalovirus, which can produce congenital abnormalities if women are infected during pregnancy.

### Herpes Simplex virus

Herpes simplex vesicules in the mouth. A type of herpes simplex virus is manifested in painful oropharyngeal infections inside and around the mouth, lips, pharynx, nose, face and ears. The virus remains latent in the facial nerve cells, causing the repeated appearance of vesicules.

Two types are known. The herpes virus type 1 causes feverish blisters associated with various feverish infectious diseases (colds, influenza, pneumonia). The blisters appear around the lips and in the mouth (also known as labial herpes); on the nose, face and ears, and in the buccal and pharyngeal mucous membrane. It has been possible to isolate the virus from the neuronal bodies of the facial nerve during the period between eruptions: this is its reservoir. There is no curative treatment; topical pharmacological preparations may be applied to alleviate the pain, the irritation and/or the inflammation.

The herpes simplex virus type 2 causes genital herpes. This is an increasingly widespread, sexually transmitted disease. Sometimes it is accompanied by severe headaches and fever. It begins with moderate local pruritus, followed by the progressive eruption of vesicules. These burst, form scabs and eventually dry. This entire process can last from 1 to 3 weeks. New vesicular eruptions often appear while the previous eruption is drying. Another transmission pathway is the neonatal route: the neonate of a sick mother is infected as it passes through the birth canal, contracting the systemic disease, which is usually fatal. These children have to be born by Caesarean section on account of this serious risk. Genital herpes has been treated topically since 1982 and by a systemic treatment since 1984.

The herpes virus type 2 causes cancer of the cervix (neck of the uterus): the viruses lodge in the cells of the mucous membrane and eventually, years later, cause transformation of these cells, which is sometimes cancerous. The viruses can also infect the central nervous system, particularly in patients who are debilitated or have compromised immunity, such as those suffering from cancer, and this causes serious encephalitis. Early treatment can prevent death or serious cerebral consequences.

### Herpes zoster virus

Vesicules caused by herpes zoster. Herpes zoster, or zona, is caused by the same virus as chickenpox. The eruption of vesicules, which is generally limited to one side of the body, occurs when the latent viral particles are reproduced in the nerves of the skin. As the virus attacks all the nerves, a pain, which is sometimes very intense, can last for many months after the vesicules have cured. Fortunately, herpes zoster is not common, the elderly and immuno-compromised patients being affected most frequently and seriously.

Recurrence of the varicella-zoster virus, which was not eradicated in its entirety by the immune system during infantile chickenpox and remained lodged in the nerve ganglia; the virus is reactivated in situations of immunodeficiency and causes the infection known as herpes zoster or zona. The skin that is enervated through the nerve containing the virus suffers an eruption of vesicules, accompanied by intense pain and increased sensitivity. Initially, the vesicules are full of a clear liquid, which then becomes cloudy, and they finally burst and form scabs that dry after 5 to 10 days. The pain produced by the herpes zoster virus may be intense and last several weeks. After recovery, neuralgia may persist in the affected area. High-dose treatment may reduce the symptoms, and an analgesic treatment is also applied. Serious cases are treated with corticoids (cortisone). Persistent neuralgia is treated by blocking the nerve trunk or by surgery.

In patients undergoing chemotherapy for neoplastic diseases, the development of herpes zoster may be fatal. Vaccines for children being treated for leukaemia are being tested in Japan; early results are promising.

Herpes infection of the eye, known as dendritic keratitis, can cause irreversible lesions of the cornea.

### Types of virus

Picornaviruses are a group of viruses forming the Picomaviridae family, of which the genome is formed by single-stranded (RNA) acid. They are very small, icosahedral viruses (bodies having regular surfaces in their topological presentation), which is why they have the name of picornavirus from 'pico' meaning small amount. They cause many gastrointestinal, respiratory and cutaneous diseases, as well as diseases of the nervous system and the mucous membranes. They include four genera, Enterovirus, Rhinovirus.

### Rhabdovirus

Group of viruses forming the Rhabdoviridae family, of which the genome is formed by linear single-stranded (RNA) acid. They infect vertebrates, invertebrates and plants. This family includes the rabies virus and other related viruses of the Lyssavirus and bovine popular stomatitis genera.

The Adenovirus; Arenavirus; Coronavirus; Filovirus; Hantavirus; Hepadnavirus; Herpesvirus; Orthomyxovirus; and Paramyxovirus are similar to the foregoing.

There are some viruses, discovered in the 1970s, which cause severe fevers and are found, in particular, in tropical regions. These include the Ebola, Marburg, Bunya, Chikungunya and Lassa viruses. Some cause death due to haemorrhaging. Dengue is a further member of this family of viruses, which has been known for some time but which has spread to Mexico and the Caribbean region in recent years. Diseases in this group are rare, but are nevertheless dangerous.

Analysis of the immune response to a variety of infectious agents has been limited because it has sometimes been difficult to cultivate pathogens in adequate quantities to allow the isolation of surface antigens of significant cells. The arrival of molecular cloning has reversed some of these limitations by providing a medium where the genetic products of pathogenic agents may be expressed in virtually unlimited quantities in a non-pathogenic form. The surface antigens of viruses such as the influenza virus, foot and mouth infections, hepatitis, papularstomatitus virus, rabies virus and herpes simplex virus have been expressed in E. coli and in S. cerevisae, and they promise to provide subsets of improved vaccines in the future. However, it is obvious that the expression of surface antigens in inferior organisms is not entirely satisfactory and that potentially determining significant antigens may be lost through an incomplete process, in other words proteolysis, glycosylation or by denaturation during purification of the genetic product.

This is true in the case of protein membranes which, owing to their nature as a hydrophobic transmembrane, tend to aggregate and make themselves insoluble when they are expressed in cloned E. coli genes, which encode protein membranes that may be expressed in mammalian cells when the host cell provides the factors required for the innate process, doubling of the polypeptide, and incorporation within the cell membrane. While these studies demonstrate that the membrane proteins may be expressed at the surface of a recombinant host cell and, for example, that a truncated membrane protein which lacks the carboxyhydrophobic terminal can slowly secrete from the host cell while combining therewith, it is not obvious that any of the expressed binding membrane proteins or the secreted truncated protein will be capable of acting by releasing antibodies which are effective against the pathogen from which the protein is derived.

US Patent 6,180,357, granted on 30 June 2001, protects and discloses a method of producing patient-specific anti-cancer antibodies, in other words customised antibodies which may be used for therapeutic and diagnostic purposes. The antibodies are produced from tissue cells from a tumour from a particular patient, and are selected on the basis of his cancer cell cytotoxicity and simultaneous lack of toxicity of non-cancerous cells. The antibodies may be used to detect and diagnose the cancer and may be used to treat tumour metastases. Anti-cancer antibodies may be conjugated to red blood cells obtained from the patient and may be reinjected to treat the metastases based upon the recognition that metastatic cancers are usually well vascularised and the delivery of anti-cancer antibodies by red blood cells can have the effect of concentrating the antibodies at the site of the tumour. The antibodies are designed to identify a subset of antibodies which express an enhanced degree of cytotoxicity directed toward cancerous cells while simultaneously being non-toxic to non-cancerous cells of said individual's tissue sample, so said subset of antibodies defines a group of customised anti-cancer antibodies.

Historically, the use of polyclonal antibodies had little success in the treatment of human cancers. Lymphomas and leukaemias have been treated with human plasma, but the response has been poor. In addition, reproduction was low and did not give additional benefits in comparison with chemotherapy. Solid cancerous tumours, melanomas and carcinomas of renal cells have also been treated with human blood, chimpanzee serum, human plasma or horse serum, with a corresponding unpredictable result and ineffective results. Numerous clinical trials have been carried out with monoclonal antibodies, antibodies against targets of glycoproteins and glycolipids, and all have had at most a 30% to 50% success rate in the cases treated.

US Patent 6,348,309, granted on 19 February 2002 to Mohr *et al*., discloses and protects a process for inactivating viruses in blood and blood products in which a phenothiazine dye is added and then irradiated by light. The use of a very small concentration of phenothiazine dye avoids any adverse effects on the blood or blood product, and, after irradiation, the dye may be separated from the blood or blood products by a dye absorbing agent. The dye concentration is approximately 0.5 mu.M to 2 mu.M, and the blood is irradiated with light having a wavelength in the range of the absorption peak of said phenothiazine dye for a period of approximately 30 minutes.

US Patent 6,254,873, granted on 3 July 2001, discloses and protects a vaccine for the dengue virus, which protects humans from dengue disease, the vaccine containing a dengue virus which has been purified and inactivated by the method involving the following steps: propagating the virus in a cell culture, harvesting said virus from said cells, concentrating said virus, purifying said virus such that it is free from cell culture proteins and DNA, inactivating said virus and adding a suitable adjuvant in a pharmaceutically acceptable amount.

US Patent 6,239,099, granted on 29 May 2001, discloses and protects a method of treating viral infections, the invention providing a method and compositions for treating these infections in mammals, comprising the administration of an effective amount of modified C-reactive proteins (CRP), r.sub.m CRP or mutant CRP. The viral infections treated are caused by viruses such as Herpes, papilloma, Epstein Barr and Retroviridae viruses. In particular, it has been found that they are effective in treating retroviral infections. The invention also provides a method of neutralising a virus in the blood which is to be used for transfusions by adding the modified CRP to the blood prior to the transfusion. The CRPs bind to the viruses, contributing to phagocytosis of the virus or, in other words, are capable of neutralising the virus.

US Patent 6,204,058, granted on 20 March 2001, discloses and protects a treatment for so-called autoimmune diseases by the administration of a vaccine to human patients. The vaccine comprises an aliquot of the patient's blood containing, *inter alia,* leukocytes having upregulated expression of various cell surface markers and lymphocytes containing reduced amounts of certain stress proteins. It is produced by subjecting the blood aliquot, extracorporeally, to specific stressors, namely oxidising agents, ultraviolet light radiation and elevated temperature (37 to 55°C). The method involves extracting an aliquot of blood from the patient, modifying said aliquot extracorporeally by subjecting it in an immune system to an atmosphere of ozone gas and ultraviolet radiation simultaneously so as to increase the leukocytes exhibiting morphology similar to apoptosis in said aliquot. The aliquot size is 0.01 to 400 ml. The immune response to the antigen is excessive owing to the interaction of an antigen with receptors of predetermined specificity to specific lymphocytes. It is believed that, in an early stage of immune system development, those lymphocytes with receptors which- recognise autoantigens are recognised and eliminated from the body's system by a process of suppression. Alternatively, these autoreactive lymphocytes may be controlled by suppression of their activity. The immune system of a normal individual is capable of identifying and reacting against a family of proteins which are largely preserved in nature and have a similar structure in all living organisms. It is known that so-called autoimmune diseases, or at least some of them, are probably associated with inappropriate control of the autoimmune response. It will be possible to control an inappropriate autoimmune response by stimulating the body's natural immune control mechanism by a particular, specific method of vaccination.

US Patent 6,153,200, granted on 28 November 2000, discloses and protects a vaccine composition which is useful in inducing immune protection in a host against arthritogenic peptides involved in the pathogenesis of rheumatoid arthritis. Each vaccine composition provides an antigenic dnaJp1 peptide and optionally a further peptide fragment of the microbial dnaJ protein and/or human homologues thereof. Methods of identifying a person who is predisposed to develop rheumatoid arthritis and methods for using the vaccines are disclosed.

US Patent 6,090,599, granted on 18 July 2000, discloses and protects a treatment for viral inactivation of the red blood cells using phthalocyanines and red light. The first process involves treating the red blood cell-containing composition to inactivate an intracellular or extracellular virus, which may be present in said red blood cell-containing composition, by subjecting said red blood cell-containing composition to a virucidally effective amount of a phthalocyanine and red light, wherein the process involves (i) determining the action spectrum of said phthalocyanine for causing inactivation of said virus; (ii) determining the action spectrum of said phthalocyanine for causing the red blood cell damage; (iii) comparing (i) and (ii), and, if (i) and (ii) are not identical, determining the wavelength at which the largest favourable difference exists between (i) and (ii); (iv) providing a red light source emitting red light only in 10 nm in either direction of the wavelength determined in (iii); and then (v) subjecting said red blood cell-containing composition to said virucidally effective amount of said phthalocyanine and said provided light source. The second process involves transfusing red blood cells to a patient in need thereof by withdrawing red blood cells from a donor in whom the red blood cells have been subjected to the first process.

US Patent 6,090,387, granted on 18 July 2000, discloses and protects methods of vaccination against diseases resulting from pathogenic responses. This invention provides vaccines and means of vaccinating a vertebrate and for preventing and controlling specific T-cell mediated pathologies, including so-called autoimmune diseases and the unregulated replication of T-cells. The vaccine is composed of a T-cell receptor (TCR) or a fragment thereof corresponding to a TCR present on the surface of the T-cells mediating the pathology. The vaccine fragment may be a peptide corresponding to TCR sequences characteristic of the T-cells mediating said pathology. This peptide may bind to conventional antigens completed to MHC cells presenting antigens or to super-antigens. Means of determining appropriate amino acid sequences for these vaccines are also provided. The vaccine is administered to a vertebrate in a manner that induces an immune response directed against the TCR of pathology-mediating T-cells. This immune response down-regulates or deletes the pathogenic T-cells. The invention additionally provides specific β-chain variable regions of the T receptors, which are associated with the pathogenesis of so-called autoimmune diseases, such as rheumatoid arthritis and multiple sclerosis.

US Patent 6,066,489, granted on 23 May 2000, discloses and protects a method of treating blood-borne viral pathogens, such as HIV. A method and an apparatus for destroying blood-borne pathogens is disclosed, which uses a low intensity direct current to generate positive particles from various metals which destroy viral pathogens. A first electrode composed of silver is inserted, via a catheter, into the patient's venous system. A second electrode is placed on the exterior of the patient in the vicinity of the first electrode. A low intensity direct current is applied to the first electrode, which releases silver cations to be bonded to the virus, resulting in the denaturing of the virus.

US Patent 5,882,591, granted on 16 March 1999, discloses and protects a method and an apparatus for disinfecting biological fluids through the interaction with ozone gas, electric fields and superimposed magnets. Biological fluids, such as plasma, serum, semen, milk or blood, may be treated efficiently with ozone to inactivate certain viruses, bacteria, fungi, etc. To effect or enhance the contact between such fluids and ozone, other disinfectant or deactivating gas, the fluids are thoroughly nebulised or atomised, in other words dispersed into minute droplets. A fine rain is created through a controlled ozone atmosphere where electric and magnets fields are present. A considerable reduction in the HIV virus is observed.

US Patent 5,846,540, granted on 8 December 1998, discloses and protects immunogenic chimeras comprising nucleic acid sequences encoding endoplasmic reticulum signal sequence peptides and at least one other peptide, and their uses in vaccines and disease treatment. The invention relates to the preparation of vaccines against viruses capable of directing host organism synthesis of immunogenic chimeric proteins, which may be used as immunogens, as vaccines, or in methods of treating cancer, infectious diseases or so-called autoimmune diseases.

US Patent 5,123,901, granted on 23 June 1992, discloses and protects a method of separating pathogenic or toxic agents from a body fluid and returning it to the body. A fluid containing a preselected toxic or pathogenic agent is passed along a mixing coil with a plurality of paramagnetic beads. The paramagnets beads include a coating which selectively binds the preselected pathogenic agent. By generating a magnetic field, the paramagnetic beads having the bound pathogenic agent are magnetically separated from the fluid. In one embodiment, the method includes the separation of HIV virus or HIV-infected T-lymphocytes from blood cells. The method and the apparatus of the present invention are particularly useful for the continuous purging of a preselected pathogen from the blood of a human subject.

### Brief description of the drawings

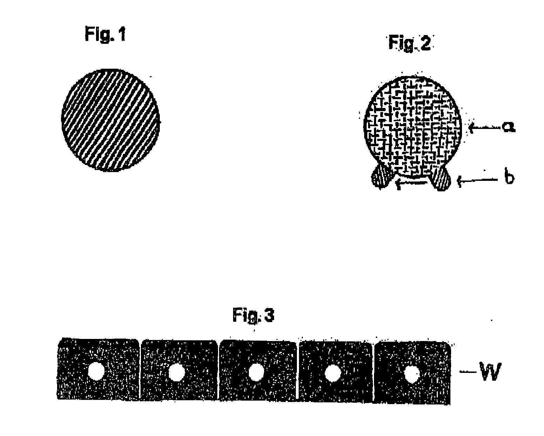

Fig. 1 shows an antigenically active germ in its entirety.
Fig. 2 shows a partially antigenic genn with (a) the antigenically inactive fraction (haptenoid) and (b) antigenically active fractions (hyperpathic plaque).
Fig. 3 shows the cells of the defence system of the organism W (endothelial reticulum)
Fig. 4 shows the defence system of the organism W: the fractions (a) do not stimulate the defence system, only fractions (b) stimulate it.
Fig. 5 shows an immune reaction where the antibodies (c) formed are incomplete and only neutralise the antigenically active fraction (b), while the fraction (a) remains free.
Fig. 6 shows an immune reaction which would be incomplete, but with a stimulus equal to one, the endothelial reticulum responds only moderately.
Fig. 7 shows the *in vitro* mixing of the haptenoid and hyperpathic plaque (PHP) with the corresponding hypogammas (HG).
Fig. 7A shows the interaction *(in vitro*) of the hyperpathic plaque (PHP)-hypogamma (HG), wherein the hapigens (HP) are attracted, precipitate, form clusters and agglutinate the hapigen by means of its hyperpathic plaque, the hyperpathic plaque remaining active in the vicinity of the cells.
Fig. 8 shows that the hapigen is only partially neutralised, and that the unneutralised fraction irritates, injures and finally destroys the cells which are in contact therewith.
Fig. 9 shows a complete immune reaction where, to be antigenically active, all the fractions of the body M cause the formation of antibodies with each and every one of them; this leads to the production of complete antibodies (C) and therefore to a state of complete immunity.
Fig. 10 shows the parts of a hapigen with the haptenoid fraction (inactive fractions a, d) and the active fraction or hyperpathic plaque (b).
Fig. 11 shows the hypogamma hapigen complex, which will be converted into an antigen, in other words with all of its fractions antigenically active, by the process.
Fig. 12 shows the antigen introduced into an organism, causing the formation therein of complete antibodies c, which neutralise all the fractions a, b, d.

### Brief description of the invention

An object of the present invention is to provide a process for increasing the production of complete antibodies capable of eliminating the pathogenic agent.

A further object of the present invention is to provide a process for preparing a customised medication for the patient's specific disorder.

Yet a further object of the present invention is to customise the therapy of each patient specifically for the treatment of his disease by means of complete antibodies.

Yet a further object of the present invention is to provide those who practise medicine with a method which is medically appropriate for treating the afflictions of patients in a specific customised manner.

And yet a further object of the present invention is to provide therapy which will be applicable to any patient and does not generate side effects.

Yet a further object of the present invention is to provide a method and product obtained to increase the statistics for the curing of diseases such as herpes, AIDS, so-called autoimmune diseases, etc.

It is an object of the present invention to provide a new autovaccine, which is useful in alleviating the symptoms of at least one of the so-called autoimmune diseases.

A further object of the present invention is to provide a new method for preparing an autovaccine.

A further object of the present invention is to provide a method of enabling the immunological system to recognise the pathogenic agent in its entirety in order to prepare the corresponding complete antibodies.

A further object of the present invention is to provide a medication prepared for the method disclosed herein for treating so-called autoimmune diseases.

A further object of the present invention involves extracting from the patient a quantity of 1 to 10 tenths ml of blood which is brought into contact, extracorporeally, with a quantity, which is 10 to 100 times greater, of an isotonic liquid that does not denature it. The treated blood is then administered to the patient in quantities that are regulated in terms of volume and time.

A further object of the present invention is to cure so-called autoimmune diseases by the administration of vaccines prepared with minimal doses, broken down as much as possible, of the same substances or elements as those which cause the disease.

### Detailed description of the invention

A new immunological element is proposed, which is neither a hapten nor an antigen but merely a fraction of the two. Its name, "hapigen", is composed of hapten and antigen, as it is formed by two fractions with their own personality: one fraction that is antigenically active and is known as the hyperpathic plaque and the other fraction that does not stimulate the immunological system as it does not produce antibodies against it; this fraction is known as the "haptenoid". Although the haptenoid is bound to the hyperpathic plaque which is antigenically active, it remains antigenically inactive and does not stimulate the immunological system as the immunological system only neutralises the active fraction or the hyperpathic plaque and does not provide any defence against the haptenoid which remains free with the ability to irritate the region where it is located. The antibody produced against the natural hapigen is known as "hypogamma" (not antibody), because it is cannot neutralise the complete hapigen.

A new immunological element is defined, which is neither hapten nor antigen, and is known as "hapigen" because it has the characteristics of both; one fraction of the hapigen is capable of stimulating the defence system of the organism, in the same way as the antigen, but the remainder behaves as a hapten, which is immunologically inactive in its natural state. The antigenically active fraction of the hapigen is called the hyperpathic plaque and the fraction which is inactive is called the haptenoid.

The hapigen, which may be a virus, microbe, parasite or inert substance, in response to the endothelial reticulum of the host, by means of its hyperpathic plaque, leads to the production of elements which have been considered as "antibodies". However, as antibodies have to neutralise the stimuli which cause the production thereof, it is misleading to call these elements antibodies, since this result is not achieved.

The existence of these "pseudo antibodies" is clear in numerous diseases such as AIDS, rheumatic fever, Malta fever, chronic salmonellosis, infectious arthritis, etc., the presence thereof, although in large numbers, not checking the disease or leading to a cure.

The real reason why these misnamed antibodies are useless in curing the corresponding disease is because they are merely a product of the response of the organism's defence system to stimulation by the hyperpathic plaque of the hapigen. Those elements which are incapable of neutralising the pathogenic agent are known as "hypogammas" (inadequate globulins). The hypogammas are capable of agglutinating their hapigens (*in vitro*, hence the positive serological reactions in AIDS, brucellosis, etc); however, this is not why the hapigens lose their pathogenic power, and this is evidence that it may be obtained by inoculation of receptive animals in which the specific disorder is generated.

Attacks by hapigens produce allergic reactions and states of autoimmunity, as they are currently called, though this designation is tending to change because autoimmunity does not exist, as explained in the present invention. Allergies as well as autoimmune diseases are caused by hapigens, which deceive the organism's defence system with their hyperpathic plaque and do not give it the opportunity to produce complete antibodies against the entire hapigen. Therefore, it is necessary to convert the hapigen into a complete antigen, which completely stimulates the organism's immunological system.

When hapigens are transformed into antigens and injected into the sick organism, the immunological system responds immediately and correctly by producing complete antibodies against the pathogenic agent, which will cure the sick organism. In fact, when a sick organism recovers, with its immunological system functioning normally and without having received anti-allergy drugs or desensitising vaccines, the allergies and the autoimmunity to thus named diseases disappear.

To transform the hapigens into complete antigens, the hyperpathic plaque is first covered with the corresponding hypogamma and is then transformed into an antigen. The hapigen and the hypogammas are obtained from previously inoculated animals, from cultures or from the patients themselves, the hyperpathic plaque of the hapigen is combined with the specific hypogamma, its electric charge and its ambient medium are modified so that it becomes antigenically active in its entirety, it is deposited in a medium which does not denature it and does not prevent its vital functions in the case of bacteria or viruses. The hyperpathic plaque of the hapigen is thus covered with its respective hypogamma and transformed into an antigen.

The name of "antibody" should be reserved exclusively for those elements that are produced by complete antigens or genuine antigens, such as the smallpox virus or the measles virus, whose attack on the organism actually leads to the formation of complete antibodies with which the invaded organism can cure itself and also reach a state of genuine immunity that protects it against new attacks by the same antigen.

Ignorance of the existence of hypogammas gave rise to the belief that some of the organism's defences could be harmful to it; it was erroneously believed that they were complete antibodies and only hypogammas neutralise only the active portion of the hapigen, and they only served the hapigen by retaining it and agglutinating it in the cells of different tissues, in which it caused a wide variety of disorders. Although more hypogamma-producing reactions take place in the sick organism, a greater quantity of pathogenic elements (hapigens) will agglutinate and the effects or lesions will be greater in the cells or tissues with which they make contact.

Once the hapigen had been discovered and defined, it was possible to convert it into an antigen and to develop a method for preparing curative vaccines for a large number of diseases which were difficult or even impossible to cure by conventional therapeutic methods, including the most potent modem antibiotics which involved genetic engineering. The list of diseases which can be cured include: Malta fever (caused by any type of brucella in any period of evolution), gonococcal and streptococcal arthritis; chronic salmonellosis which does not respond to conventional antibiotics and vaccines; rheumatic fever; thrombocytopenic purpura; scleroderma; sexual herpes; AIDS; etc. They are all diseases caused by hapigens that are transformed into antigens, so the organism produces complete antibodies that neutralise the hapigen, wherever it is located (intracellular or extracellular).

In the case of brucellosis caused by Brucella melitensis, and AIDS, the sick person's defence system returns to normal function after having eliminated the viruses and microbes.

This method take the complete antigen and considers the vital, metabolic, reproductive or growth aspect as a whole, not just a topological aspect of the form, membrane, protoplasm or nucleus.

Different types of vaccine are not described here, but merely a single method of preparing curative vaccines, in which the agent that caused the disorder (brucella in Malta fever, Eberth bacilli in typhoid, paratyphi A and B in paratyphoid fever, HIV viruses in AIDS, type 2 viruses in sexual herpes, etc.) is always used in a specific form.

Investigations have been carried out to discover their efficacy and innocuousness, not only for their recipients but also for their handlers. Patients representative of each disease or disorder (Malta fever, typhoid, paratyphoid, typhus, gonococcal and streptococcal arthritis, herpes type 2) were taken and were administered the specific vaccine corresponding to their disease; each group was compared with another similar group of ten patients having the same disorder, who received only physiological serum as a placebo in the same doses and at the same time intervals as administration of the vaccines to the other group.

The result was a complete success, as all patients who received the vaccines were cured: in a period of between 4 and 7 months in the case of brucellosis, typhoid, paratyphoid, typhus and in a period of between 12 and 18 months in the case of those suffering from sexual herpes.

Those who received a placebo remained ill, but were then given the vaccine and cured.

Each patient has a unique disorder according to his human individuality. Common therapy treats all patients with the same type of disorder at the same stage in the same way.

With the advent of monoclonal antibodies, the possibility of developing customised methods of therapy became more realistic, since each antibody can be directed to a single epitope. It is thus possible to produce a combination of antibodies that are directed to the constellation of epitopes that uniquely define a particular individual's tumour. Having recognised that the significant difference between cancerous cells and normal cells is that cancerous cells contain antigens that have specificity (haptenoid: non-reactive part of a hapigen) to transform cells, the scientific community is hopeful that monoclonal antibodies (hypogammas: antibodies which neutralise the active part of the hapigen) can be designed to specifically target transformed cells by binding specifically to these cancer antigens; thus giving rise to the belief that monoclonal antibodies have this magic property of acting as "magic bullets" to eliminate cancer cells or cells infected with some other disorder. At present, cancer patients have few options for treatment and those which they do have will not necessarily improve their personal situation.

Think of an experimental doctor, a solitary gentleman, who feels that he has reached the bottom of things and with whom one can get deeper and deeper. This man is a leader who has a monopoly on time in the field of scientific adventure, with clever suggestions. This gentleman opens up a new field and is capable of moving in it and leading us through and along it, in other words is taking us inside. As a result, he can tell us about many very interesting things that happen within strange phenomena from the unfamiliar field. In addition, the most important point is the large number of possible technical applications in this field.

We are dealing with the problem of establishing, manipulating and controlling phenomena which occur in this small field and in which life and its transformations are present. We are talking about a biological system.

A biological system deals with things on a very small scale and makes one think that it should be possible to manipulate it in order to produce something on this excessively small scale. The majority of cells are very small and insignificant, but extremely active. They can produce various substances and supply information. Just consider the possibility that, on this small scale, we could order whatever we want and that we could produce an object or a mode of operation at this level. This is the case with hapigens and hypo gammas, which complement one another to transform a haptenoid into a complete antigen, where the haptenoid is composed of the active part (hyperpathic plaque) and the non-reactive part.

Up until now, the fight against allergies has been an uneven fight, as when fighting an unknown enemy whose harmful, sometimes dreadful, effects only can be perceived. This has led an infinite number of investigators to expend huge efforts in an attempt to neutralise the effects, or at least mitigate the power, of such a powerful adversary. The power does not reside in their energy or in the speed of their effects, but in the fact that they are unknown.

Many patients, particularly those who have not been able to obtain a cure, will not have failed to notice that their doctor is impotent against an enemy which is invincible because it is unknown. If these patients convince themselves that their illness can be reliably cured, they will display a favourable change in security and tranquillity, in the knowledge that the symptoms will diminish considerably and, for some, the discomfort will frequently disappear.

It is acknowledged here that the cause of the allergy lies in an antigen-antibody reaction, but it is not possible to find a theory to solve all the problems arising from allergic phenomena or the method of effectively combating them.

It is also considered here that all allergic phenomena are simply manifestations of reactions of incomplete immunity. They are not incomplete reactions of immunity, but complete reactions which, nevertheless, do not confer complete immunity. This type of incomplete immunity is caused in the organism by the presence of a foreign body which has or can have an antigenic capacity only in some of the fractions of its morphology; in other words; not all of the foreign body is immunologically active. This foreign body can be endowed with life, like viruses or microbes, or can be inert, like dust.

It cannot be stated that the reaction, which this class of foreign bodies causes in the invaded organism, is an incomplete reaction of immunity, because the response of the endothelial reticulum thereof is complete against the fraction or fractions of the foreign body which stimulate it and which it completely neutralises. However, we cannot say that such an immune reaction has led to a state of complete immunity, because immune means that it is completely protected and, in this case, some of the fractions of the foreign body remain unneutralised and can damage the organism; in other words it is not protected against the entire foreign body.

The stimulation of the endothelial reticulum by only a fraction of the foreign body results in the production of a specific type of antibody which, although it is sometimes capable of precipitating (partial neutralisation) *in vitro* with the complete molecule of the foreign body, can only neutralise *in vivo* the fraction of the foreign body which caused its formation. For this reason, the antibody formed is an incomplete antibody relative to the entire foreign body; therefore, it should be deemed complete for the fraction of the foreign body which led to its formation and which it neutralised completely.

The inability of the host organism to neutralise the entire foreign body enables the foreign body to remain in contact with the cells of the host tissue for a period, which may be prolonged, and this leads to irritation of the organism by the entire foreign body if it is not neutralised, isolated or eliminated. The organism fights uselessly, by means of its incomplete antibodies, against the foreign body, some of whose unneutralised morphological fractions adhere to those of the incomplete antibodies or release substances of the histamine family, and this causes cellular traumatism or allergic phenomena.

This is why allergic manifestations are due to the formation of the complex formed by the foreign body and the incomplete antibody, when this complex adheres to the organic tissue cells by means of its antigenically active fraction and the other part remains completely free and uncovered, this latter part irritating and traumatising the tissue cells in contact therewith, this cellular traumatism leading to the production or release of histamine, acetylcholine, etc. The symptoms are manifested in contractile fibres, secretory cells, neurons and nerve fibres.

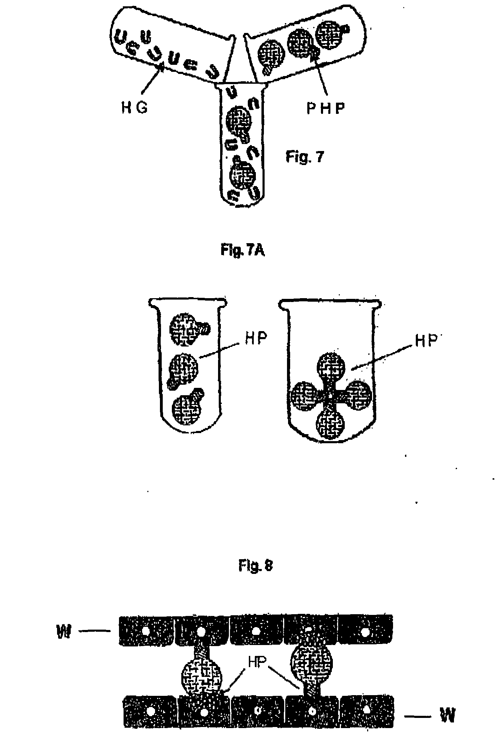

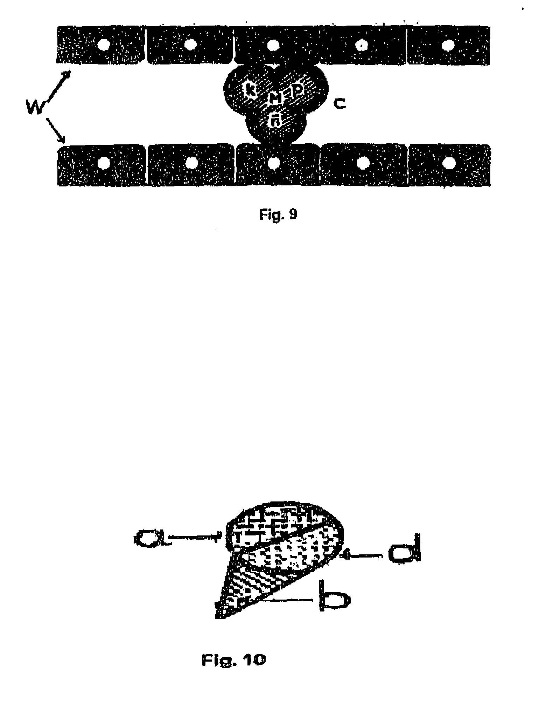

It is certain that, if a complete antibody (C, Fig. 9) were present at the traumatised sites (Fig. 8), rather than an incomplete antibody, a foreign body bound to the cell by the complete antibody would be considered as a part of the innate nature of the organism once it had been completely neutralised.

From the foregoing, an allergy is merely a phenomenon of immunity very similar to beneficial immunity in which the response is disproportionate in the number of antibodies against any pathogenic or antigenic agent. In both cases, it is common to find antibodies circulating in the organic liquids, and vaccines can be used to give the organism beneficial immunity in that the antigen can be completely neutralised. The antibodies neutralise each and every fraction or segment of said antigen (Fig. 9) as well as the metabolic products thereof. For this reason, when the antigen comes into contact with an already immune organism, the latter neutralises it before it can cause any harm.

The reason for this is that the immune organism's defence system has been taught to perform this category of defence, since its first contact with the corresponding antigen. The organism can learn to produce complete antibodies against the given antigen, and it is not because its defence system is more or less strong or because it is randomly capable of doing so, but because the antigen stimulates, with its entire being and its being morphology, the defence mechanisms of the organism; in other words, because the endothelial reticulum is stimulated by a foreign body which is immunogenically active in its entirety. The foreign body can damage the organism's immunological system as it presented to it only a stimulating part of its being, while the others remained completely freely active (Fig. 8).

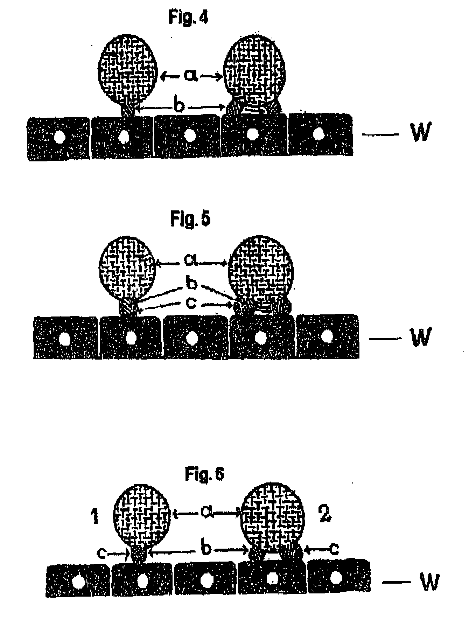

Therefore, the state of the organism that is known as the allergic state is, in reality, a state of incomplete defence (Figs. 6 and 8) caused by incomplete stimulation by a foreign body which only possesses antigenic properties in some of the fractions of its morphology and metabolic products.

There exists a substance which, owing to the simplicity of its constitution, is incapable by itself of stimulating the organism's defence system, but stimulates it when it is bound to another substance. This substance is known as hapten. The paradox or Richet's dogs will be described hereinafter, to explain the above-mentioned state of incomplete immunity or incomplete neutralisation. It also demonstrates that the organism can be taught to defend itself.

### Paradox of Richet's dogs:

A number of dogs were injected with a small dose of actinocongestin; after being injected for 2 or 3 weeks, they were given a relatively high dose of the same substance. This second dose did not poison them, despite being high, but did cause in them an intense anaphylactic shock, from which they recovered and continued to live. On the other hand, when normal dogs were injected with the second dose of the substance, without having received the first preparatory dose, they all died; this was due not to an anaphylactic shock, but to poisoning caused by the toxicity of the actinocongestin.

There are some diseases which, after having presented in the organism, never recur, for example measles, whooping cough, yellow fever, and parotiditis. There are others that recur once or often, without the time arriving when the organism is considered to be safe from the risk of contracting the disease. Influenza, colds, brucellosis, tuberculosis; syphilis, gonorrhoea, etc. stand out, with the organism seeming to acquire a certain tendency to contract the disease.

In view of the different responses of the organism's endothelial reticulum, it will be appreciated that it sometimes fights against enemies which attack it directly and leads to the generation of antibodies. At other times, however, it has to fight against microbes and organic substances which distract or divert the defensive-offensive effort of the organism, with only one part of their morphology or their metabolic products and, on the other hand, with the remaining part, they are quite free to injure or irritate; they are nourished, grow and reproduce calmly, without encountering any really effective obstacle in the organism to impede them, as they do not stimulate the endothelial reticulum of the organism with all of their being or morphology, and do not give it the opportunity to develop a state of genuine immunity against them. They make a victim of the organism by deceiving it, so a state of chronicity is developed therein.

In order to learn how to combat a live microbe, the organism has to direct its effort not only against the microbe or virus body but also against the substances which it produces, against its morphology and against its mutations, by means of which it may be combated or at least have its defensive capacity reduced. These microbes and viruses are endowed with movement and growth and are able to reproduce.

This group of dangerous germs includes those of the common cold and influenza, these germs having been difficult to understand and having led to errors, in terms of immunity, such as the belief that a vaccine prepared with dead viruses is more effective and durable than a vaccine prepared with live viruses (Dr. Salk).

Therefore, once the enemy was known, efforts were directed toward finding the method of destroying it. The method of combating such skilful enemies is to transform the hapigens into complete antigens and introduce them into the organism so that it can learn to neutralise them in their entirety.

In other words, to transform all germs and substances which, in their natural state, only partially stimulate the organism's defence system, into immunologically completely active entities so that they stimulate the immunological system with all their morphology and metabolic products.

This is a novel method of preparing vaccines, which are completely effective in all cases and are free from secondary reactions in the subjects to whom they are administered. Their action is to generate a large quantity of complete antibodies against the type of antigen used to prepare them which, before being modified, was hapigen, composed of an active fraction and an inactive fraction. This class of vaccines is effective in both curing and preventing diseases and gives the organism a state of genuine immunity against the type of antigen carried in the vaccine. This method is effective against infectious diseases, allergies, so-called autoimmune diseases, sexually transmitted diseases, etc.

The antigens and their corresponding antibodies have strong specific affinity, in other words they are strongly attracted to one another according to their specificity.

Let us assume that the pathogenic element is a substance formed by the fractions ABC, of which only fraction C is antigenically active. The organism's immune reaction leads to the production of the anti-C antibody, which is known as O and with which it covers or neutralises the fraction C that induced its formation. Even in this form, however, the foreign element continues to cause discomfort in the organism because it is not neutralised in its entirety (Fig. 8). It will be readily appreciated that it can never be completely neutralised by means of an antibody that only corresponds to one of its fractions. In fact, the defence is organised and takes place with the full capacity of the organism, which produces large amounts of antibodies O. However, the more vigorous production is, the worse the results are since a large quantity of cells with high numbers of incomplete antibodies (hypogammas), will also retain greater quantities of corresponding hapigens; for this reason, the discomforts and lesions caused by the hapigens will be more intense.

Once the stimulating fraction C has been covered, by means of the incomplete antibody (hypogamma) O, the new element ABCO is taken from the sanguineous fluid and, without being denatured, is treated in order to modify it into a complete antigen and is again administered to the organism so that, when the organism encounters it, it will perceive it in its entire morphology as a genuine antigen. Since the stimulating fraction C is covered, the organism's defence system will cease to be distracted in fighting solely against the fraction C and will begin to combat the entire foreign body, producing produce complete antibodies that are able to neutralise the foreign body ABCO in its entirety in this situation.

Healthy organisms which receive a vaccine prepared with this type of antigen react by producing anti-ABCO antibodies, as all of the incomplete antibody-hapigen (ABC+O) complex has been converted into an antigenically active organism. At a later stage, when it comes into contact with the natural hapigen ABC, since the fraction C is antigenically active, it will generate the incomplete antibody O which binds with the hapigen ABCO which, in turn, will immediately be completely neutralised by the anti-ABCO antibody which the vaccine has taught the organism to produce. Logically, the defence system of the sick organisms receiving the vaccine also begins to produce the corresponding complete antibodies, which neutralise both the antigen which is administered to them and by which the stimulating fraction is already covered, and the pathogenic agent which is in its organism, giving rise to the formation of complete antibodies. This gives the organism a biological memory which will defend it from foreign bodies ABC and ABCO.

Immune reactions, such as those described hereinbefore, involve electric charges, morphology, chemical composition and topological 3-dimensional orientation. When an organism possesses complete antibodies against a given antigen, it is able to neutralise other elements of the same class as the antigen in terms of charge, form, orientation and chemical composition, even though they are not antigenically active.

The general pattern of the above-described method corresponds to inert hapigens and live hapigens, such as brucellae, Koch-Weeks bacillus, Treponema pallidum, etc. If the body ABC is reproducing in the organism, the organism is obliged to produce the incomplete antibody O which neutralises the stimulating fraction C in such a way that it forms the complex ABCO which will be neutralised by the complete anti-ABCO antibody, since the organism has already been taught to produce it by means of the vaccine.

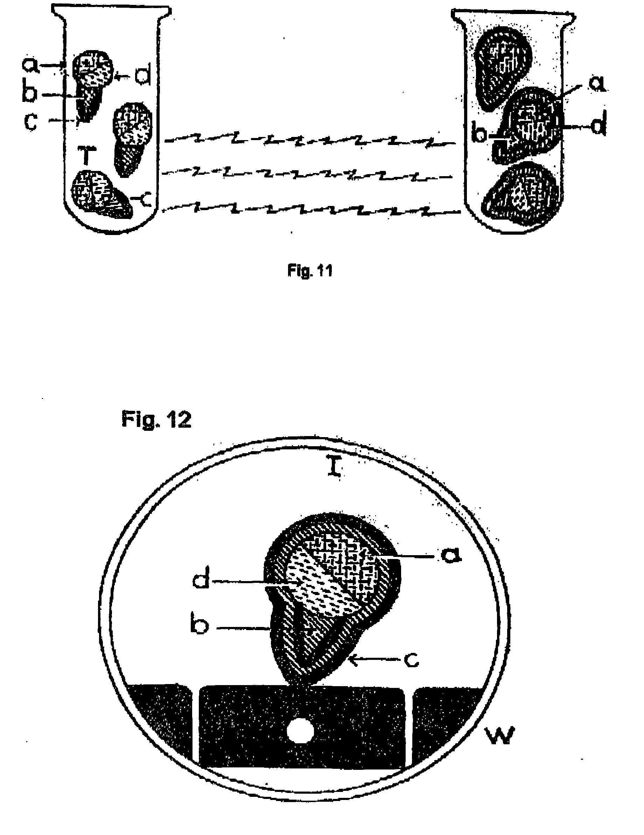

It has been stated in this application, based on scientific experiments, that hypogammas are capable of agglutinating their hapigens by acting on the hyperpathic plaque thereof (Fig. 11). However, they do not neutralise the pathogenic power of the hapigen. The injection of an isotonic solution of the agglutinated material without denaturing it into sensitive animals, causes the disease to develop as if the agglutinated hapigen had been in a culture medium. This occurs because, although the hypogamma can agglutinate the hapigen, it is incapable of removing the pathogenic power of the hapigen because it is not an antibody for the entire hapigen, so the hapigen will continue causing the disease (Fig. 8).

In view of the foregoing, it is necessary to transform the hapigen into a complete antigen so that the immunological system of the organism identifies it as an antigen and is able to produce the corresponding defences. The immunological system will be stimulated by the transformed hapigen, and the corresponding antibodies destroy it and it is eliminated to achieve the cure.

As the first step, it is necessary to cover the hyperpathic plaque with the corresponding hypogamma, and this action is carried out by the organism itself when it is stimulated by the active part of the foreign body. The physical, chemical, electrical and biological changes which are necessary, without denaturing the hapigen, then take place in the hapigen. These changes lead to a hapigen which is already transformed into an antigen and which, in its entirety, stimulates the immunological system of the organism receiving it, leading to the generation of complete antibodies which destroy the cause of the disease, so the disorder is thus cured (Fig. 12).

The method essentially consists in:
obtaining the hapigens and their corresponding hypogammas from previously inoculated animals, cultures and the patients themselves;
combining the hyperpathic plaque of the hapigen with the corresponding hypogammas;
modifying the hapigen to make it antigenically active in its entirety;
endowing it with the necessary electrical charge (polarisation);
depositing it in a solid or liquid medium which does not denature its vital functions.

During early investigations, the hapigen was injected into sensitive animals and the hypogammas required for *in vitro* coverage of the hyperpathic plaque of the hapigen causing the disease were obtained from them. This material was deposited in an isotonic liquid which did not denature it, and the necessary vaccines were prepared.

A much simpler, more economical method was based on the supposition and confirmation that the same sick organism produces its own hypogammas and also that, in their bloodstream, the hapigens find their hyperpathic plaque already covered by the corresponding hypogamma, and it was deduced and confirmed that it was not necessary to use foreign hypogammas to cover the hyperpathic plaque of the pathogenic agent, but that it was possible to use the elements produced by the patient himself, by taking them from his blood and then transforming the hapigen into a complete antigen. In this way, it is possible to obtain the curative vaccines prepared from the patient's blood, which may be applied to any hapigen of any disease from among the numerous diseases which the patient may have as well as the mutations which have occurred and the products of the toxins thereof.

To prepare the vaccine, it is merely necessary to take one unit of a sample (1 to 10 tenths of a cm³) of the patient's blood and to dilute it in a volumetric ratio of 7 to 15 cm³ of any isotonic liquid which does not denature it, for example CINa physiological serum. With this simple change of surroundings of the blood in physiological serum, which is new in physical, chemical, electrical and biological terms, any hapigen found in the patient's blood is transformed into an antigen and is capable of stimulating the immunological system to produce the complete antibodies required to destroy the disease-causing hapigen.

It is obvious that vaccines prepared in this way do not represent a danger to the person handling them or to the patients and close relatives, as they are made from the user's own blood in a very high dilution, and the only thing which this type of vaccine can cause in anyone who comes into contact with it is to teach his organism to produce complete antibodies against the hapigen or the hapigens which have been transformed into antigens that are causing the disease.

This is due to the fact that the various hapigens meet in the patient's blood and can be transformed into antigens which cause numerous diseases, such as: the brucellas (Malta fever, undulant fever, osteoarticular, visceral and meningeal diseases); syphilis, chronic gonorrhoea; whooping cough; lymphogranuloma venereum; Hodgkin's disease; allergies to household dust, rheumatism; allergies (pollen, household dust, flour); sexual herpes; rheumatoid arthritis, especially diseases caused by haemolytic beta streptococcus, golden staphylococcus and gonococcus; thrombocytopenic purpura; retroviruses such as the AIDS virus.

It should be explained that, in the case of the HIV virus, the Elisa and Western Blot reactions remain positive since these are reactions to detect antibodies and not the presence of the virus. In these patients, the T/4 and T/8 lymphocytes appear in quantities which increase daily until the number thereof reaches normal values and the proportion thereof is also normalised. In this respect, the vaccines are effective when the infected organism is still capable of reacting favourably, in other words when the proportion of CD-4 and CD-8 lymphocytes is greater than 0.30.

With this method of obtaining vaccines, it is obvious that there are many diseases which are erroneously called autoimmune diseases even though they are not.

On the other hand, Karl Landsteiner demonstrated, at the beginning of this century, that the antigen antibody reaction is so specific that, up to the position where there is some antigenically active fraction within the immunogenic molecule, it has a decisive influence on whether the antibody reacts or does not react with the antigen.

This statistical-topological dependency is taken into consideration in the present description, since the selector-detector of these dependencies is the very same immunological system of the organism. Our contribution is to consider the existence of the hapigen, haptenoid, hyperpathic plaque and hypogammas in their above-described relationship. The manner of "changing" the hapigen, which is converted into a complete antigen so that the immunological system recognises it and produces complete antibodies against it, is a further contribution.

The antigen-antibody reaction is so specific that, to provide a cure, useful antibodies have to be produced to eliminate living entities which are capable of (mutation) changing in form, size, weight, metabolic products, etc. The defence system has to be stimulated not by a hapigen but by an antigen.

For a vaccine to provide a cure (not merely prevention), it has to use as the antigen the element which causes the disease in its natural state without denaturing it and while maintaining its vital power, the pathogenic capacity, its capacity for reproduction, its motor capacity, etc., by introducing it into the organism of which the defence system collects it and learns to combat it in its entirety, with all its vital characteristics. For this purpose, when living organisms are to be combated, the vaccine has to carry the antigen without losing vital organic properties thereof (merely eliminating its capacity to cause damage), and infected organisms have to learn to combat living entities and not mummies.

A significant characteristic of the present invention is that the blood with which the autovaccine is prepared is obtained specifically from a patient suffering from the so-called autoimmune disease. The antigens which form the basis of the autovaccine are originally found in the patient's own blood. Foreign antigens are not added; the effective amount of antigens is present in the patient's own blood, and they are modified by the process of preparing the autovaccine, which uses the patient's own blood as the raw material for preparation of the vaccine. The blood sample, which is diluted as much as possible, is left to rest, is then agitated and the appropriate dose taken, in accordance with the following instructions:
1. A sample (1 to 10 tenths ml) is taken and diluted in 1 to 10 ml of isotonic liquid, which may be CINa physiological serum, and kept in a cool, dry place while avoiding interaction with ultraviolet light.
2. The first dilution is made in 1 to 10 ml of isotonic liquid and the mixture is agitated.
3. For the second dilution, the mixture is agitated and a volume of 1 to 50 tenths ml is taken, and diluted in an isotonic solution of 10 to 100 ml.
4. The flask of the first dilution is taken and the same process is continued.

Subcutaneous injections are administered 3 times a week, the dose beginning with 10 units from a 100 unit insulin syringe, increasing by 2 units at each administration for 6 weeks until 30 units are reached. Each dosing flask is calculated for 6 weeks.

The treatment may last from 6 weeks to 3 years.

Disorders of the immunological system, which may be treated in accordance with the invention, include rheumatoid arthritis, scleroderma, lupus, multiple sclerosis, psoriasis and other infectious and inflammatory disorders.

Once the vaccine has been prepared, the organism is injected in increasing doses which begin with 10 units in a 100 unit insulin syringe, the vaccine being administered 3 times per week for 6 weeks. A flask contains sufficient rations for 6 weeks; a unit is increased after each flask until a dose of 30 units is achieved.

For example, for sexual herpes, the treatment is from 1 to 2 years; for typhus, typhoid and paratyphoid, the treatment is for 4 to 5 months; for brucellosis (abortus, mellitus, suis, Malta fever) the treatment is for 3 to 4 months.

The present description is not limiting but descriptive, limitations being given only by the appended claims.

## Claims

1. Method of transforming the hapigens of an organism causing diseases known as autoimmune diseases into complete antigens, which comprises:
extracting a sample of blood from the patient suffering from the disease;
mixing the extracted blood extracorporeally *(in vitro*) by subjecting it to maximum dilution, of 10 times its volume, with an isotonic liquid which does not denature it and favourably changes its physical, chemical, biological and electrical properties so that the body's immunological system identifies it as a complete antigen and creates complete antibodies;
removing the settled residues from the mixture and preparing doses for 6 weeks;
for the second dose, taking a sample from the previously prepared dose and subjecting it to greater dilution, in the same proportion of 10 times its volume, to prepare the second dose for the following 6 weeks, and so on;
injecting the patient suffering from the disease with a dose of 10 units of the prepared sample, the dose being increased by 2 units in each period of administration.

2. Method of transforming the hapigens of an organism causing diseases known as autoimmune diseases into complete antigens according to claim 1, **characterised in that** the sample extracted from the patient has a volume of 1 to 10 tenths ml of blood.

3. Method of transforming the hapigens of an organism causing diseases known as autoimmune diseases into complete antigens according to claim 1, further **characterised in that** the sample is diluted as much as possible in physiological serum which does not denature the blood sample obtained and is agitated in an ultraviolet light-free location to mix it.

4. Method of transforming the hapigens of an organism causing diseases known as autoimmune diseases into complete antigens according to claim 1, further **characterised in that** the maximum dilution in physiological serum is effected in a proportion of 1 volume of blood to 10 volumes of physiological serum, and the substances are agitated to produce a uniform mixture.

5. Method of transforming the hapigens of an organism causing diseases known as autoimmune diseases into complete antigens according to claim 1, further **characterised in that** the dilution is left to rest, the haptenoids combine with the hypogammas by means of their hyperpathic plaque, its specific weight is increased and the hapigens settle as they are formed.

6. Method of transforming the hapigens of an organism causing diseases known as autoimmune diseases into complete antigens according to claim 1, further **characterised in that** the settled hapigens are extracted from the mixture and suitable doses are prepared for administration periods of 6 weeks.

7. Method of transforming the hapigens of an organism causing diseases known as autoimmune diseases into complete antigens according to claim 1, further **characterised in that** the doses are of complete antigens.

8. Method of transforming the hapigens of an organism causing diseases known as autoimmune diseases into complete antigens according to claim 1, further **characterised in that** the periodic doses of complete antigens of 10, 12, 14, 16, ... 30 units are injected from the 100 unit insulin syringe and the organism responds by creating complete antibodies which neutralise the complete antigen.

9. Use of the product obtained by the method of transforming the hapigens of an organism causing diseases known as autoimmune diseases into complete antigens for producing a customised medication for the treatment of autoimmune diseases.

10. Use of the product obtained by the method of transforming the hapigens of an organism causing diseases known as autoimmune diseases into complete antigens according to claim 9, further **characterised in that** it is used to produce a customised medication for treating Malta fever.

11. Use of the product obtained by the method of transforming the hapigens of an organism, causing diseases known as autoimmune diseases into complete antigens according to claim 9, further **characterised in that** it is used to produce a customised medication for treating the disease of chronic gonorrhoea.

12. Use of the product obtained by the method of transforming the hapigens of an organism causing diseases known as autoimmune diseases into complete antigens according to claim 9, further **characterised in that** it is used to produce a customised medication for treating the disease of whooping cough.

13. Use of the product obtained by the method of transforming the hapigens of an organism causing diseases known as autoimmune diseases into complete antigens according to claim 9, further **characterised in that** it is used to produce a customised medication for treating the disease of allergy to household dust.

14. Use of the product obtained by the method of transforming the hapigens of an organism causing diseases known as autoimmune diseases into complete antigens according to claim 9, further **characterised in that** it is used to produce a customised medication for treating the disease of vitiligo.

15. Use of the product obtained by the method of transforming the hapigens of an organism causing diseases known as autoimmune diseases into complete antigens according to claim 9, further **characterised in that** it is used to produce a customised medication for treating the disease of rheumatism.

16. Use of the product obtained by the method of transforming the hapigens of an organism causing diseases known as autoimmune diseases into complete antigens according to claim 9, further **characterised in that** it is used to produce a customised medication for treating the disease of rheumatic fever.

17. Use of the product obtained by the method of transforming the hapigens of an organism causing diseases known as autoimmune diseases into complete antigens according to claim 9, further **characterised in that** it is used to produce a customised medication for treating the disease of rheumatoid arthritis.

18. Use of the product obtained by the method of transforming the hapigens of an organism causing diseases known as autoimmune diseases into complete antigens according to claim 9, further **characterised in that** it is used to produce a customised medication for treating the disease of herpes type 2.

19. Use of the product obtained by the method of transforming the hapigens of an organism causing diseases known as autoimmune diseases into complete antigens according to claim 9, further **characterised in that** it is used to produce a customised medication for treating retrovirus and AIDS virus diseases.

20. Use of the product obtained by the method of transforming the hapigens of an organism causing diseases known as autoimmune diseases into complete antigens according to claim 9, further **characterised in that** it is used to produce a customised medication for treating the diseases caused by streptococcal and staphylococcal infections.
